(19) Europäisches Patentamt
European Patent Office
Office européen des brevets

(11) **EP 3 588 081 B1**

(12) **EUROPÄISCHE PATENTSCHRIFT**

(45) Veröffentlichungstag und Bekanntmachung des Hinweises auf die Patenterteilung:
**07.06.2023 Patentblatt 2023/23**

(21) Anmeldenummer: **18179315.9**

(22) Anmeldetag: **22.06.2018**

(51) Internationale Patentklassifikation (IPC):
**G01N 33/00** (2006.01)     **G01N 1/40** (2006.01)

(52) Gemeinsame Patentklassifikation (CPC):
**G01N 33/0047; G01N 1/405; G01N 33/0006**

(54) **VERFAHREN UND VORRICHTUNG ZUM KALIBRIEREN EINES FLUIDDETEKTORS MIT PRÄKONZENTRATOR**

DEVICE AND METHOD FOR CALIBRATING A FLUID DETECTOR WITH PRE-CONCENTRATOR

PROCÉDÉ ET DISPOSITIF D'ÉTALONNAGE D'UN DÉTECTEUR DE FLUIDE COMPRENNANT UN PRÉCONCENTRATEUR

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**01.01.2020 Patentblatt 2020/01**

(73) Patentinhaber:
• **Universität des Saarlandes**
  **66123 Saarbrücken (DE)**
• **3S GmbH**
  **66115 Saarbrücken (DE)**

(72) Erfinder:
• **Sauerwald, Tilman**
  **66123 Saarbrücken (DE)**
• **Schütze, Andreas**
  **St. Ingbert (DE)**
• **Schultealbert, Caroline**
  **66111 Saarbrücken (DE)**
• **Brieger, Oliver**
  **66111 Saarbrücken (DE)**
• **Diener, Robin**
  **66111 Saarbrücken (DE)**
• **Leidinger, Martin**
  **66571 Eppelborn (DE)**
• **Conrad, Thorsten**
  **66271 Bliesransbach (DE)**
• **Reimringer, Wolfhard**
  **66780 Hemmersdorf (DE)**

(74) Vertreter: **Banse & Steglich**
**Patentanwälte PartmbB**
**Patentanwaltskanzlei**
**Herzog-Heinrich-Straße 23**
**80336 München (DE)**

(56) Entgegenhaltungen:
**US-A- 5 469 369     US-B1- 6 902 701**

• **LEIDINGER MARTIN ET AL: "Integrated pre-concentrator gas sensor microsystem for ppb level benzene detection", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, Bd. 236, 14. April 2016 (2016-04-14), Seiten 988-996, XP029700025, ISSN: 0925-4005, DOI: 10.1016/J.SNB.2016.04.064**
• **HO C K ET AL: "Integrated chemiresistor sensors with preconcentrators for monitoring volatile organic compounds in water", 2005 WORLD WATER AND ENVIRONMENTAL RESOURCES CONGRESS 20050515 TO 20050519 ANCHORAGE, AK - PROCEEDINGS OF THE 2005 WORLD WATER AND ENVIRONMENTAL RESOURCES CONGRESS, AMERICAN SOCIETY OF CIVIL ENGINEERS, US, 1. Januar 2005 (2005-01-01), Seiten 273-280, XP008173602, DOI: 10.1061/40792(173)273 ISBN: 978-1-60423-627-9**

## Beschreibung

Technisches Gebiet

Die vorliegende Erfindung betrifft Fluiddetektoren zum Detektieren, Identifizieren und Quantifizieren von zu detektierenden Stoffen, insbesondere von flüchtigen organischen Stoffen (VOC: Volatile Organic Compound) in einem Trägerfluid, wie beispielsweise Luft, Wasser und dergleichen. Weiterhin betrifft die vorliegende Erfindung ein Verfahren zum Kalibrieren eines Fluiddetektors mit Präkonzentrator, insbesondere in mikrosystemischer Aufbauweise.

Technischer Hintergrund

Flüchtige organische Stoffe werden in verschiedenen Umgebungsbereichen durch künstliche Materialien in die Umgebungsluft abgegeben. Insbesondere in Gebäuden können sich diese in der Luft anreichern und für Personen schädlich sein. Abhängig von dem jeweiligen Stoff können sogar sich geringe Konzentrationen des Stoffes gesundheitlich auswirken und beispielsweise zum Auftreten eines Sick-Building-Syndroms führen. Beispiele für schädliche Stoffe sind Formaldehyd, Benzol und Naphthalin, die bereits in Konzentrationen von ppb oder sub-ppb relevant sein können.

Um die Exposition von Personen mit flüchtigen organischen Stoffen zu überwachen, können Fluiddetektoren verwendet werden, die eine ausreichende Genauigkeit und Selektivität für bestimmte flüchtige organische Stoffe haben.

Verschiedene Arten von Gassensoren sind aus dem Stand der Technik bekannt, die insbesondere Sensoren nutzen, deren Messprinzip auf der Verwendung von Metalloxid-Elementen, organischen Halbleiterelementen oder gasempfindlichen Feldeffekttransistoren sowie von Sensoren basierend auf elektrochemischen, optischen, kalorimetrischen, infrarotspektroskopischen oder gaschromatographischen Messverfahren basieren. Insbesondere Metalloxide, organische Halbleiter sowie gasempfindliche Feldeffekttransistoren (Gas-FET) bieten eine hohe Empfindlichkeit bezüglich flüchtiger organischer Stoffe.

Jedoch ist bei solchen Detektoren die Selektivität in der Regel schlecht, so dass diese Sensoren in der Regel nicht geeignet sind, bestimmte schädliche flüchtige organische Stoffe in Luft zu detektieren, da auch viele natürliche flüchtige organische Stoffe bereits durch Lebewesen abgegeben werden und so das Messergebnis verfälscht wird. Somit ist es in der Regel nicht möglich, in Räumen solche auf Halbleitern basierenden Detektoren einzusetzen.

Es ist allgemein bekannt, die Selektivität von Gassensoren durch Kombination mit einem Präkonzentrator zu erhöhen, der eine gute Selektivität aufweist und ausgebildet ist, nur bestimmte flüchtige organische Stoffe zu sorbieren und anzureichern. Die sorbierten Stoffe können desorbiert werden, insbesondere durch Erhitzen des Präkonzentrators, so dass die Konzentration des bestimmten sorbierten Stoffes in der den Präkonzentrator umgebenden Luft erhöht werden kann.

Bei Verwenden eines Präkonzentrators zum Erhöhen der Selektivität eines Fluiddetektors müssen Messzyklen mit einer Sorptionsphase, während der der Präkonzentrator zur Anreicherung eines bestimmten ausgewählten Stoffes betrieben wird, und eine Messphase, in der der Präkonzentrator aktiviert wird, um den zuvor angereicherten sorbierten Stoff freizusetzen, um so dessen Konzentration in einem Trägerfluid über die herrschende Konzentration in der Umgebung hinaus für die Messung durch den Gassensor zu erhöhen.

Beispielsweise ist aus der Druckschrift US 6,902,701 B1 eine Fluiddetektionseinrichtung bekannt, die eine Kombination aus einem Präkonzentrator für die Sorption von bestimmten flüchtigen organischen Stoffen und ein oder mehrere Chemiresistoren aufweist. Die Chemiresistoren dienen dazu, in einer Messphase die zuvor während einer Sorptionsphase angereicherten und anschließend abgegebenen flüchtigen organischen Stoffe zu detektieren. Die Anordnung des Präkonzentrators und des Chemiresistors ist in einer mikrosystemtechnisch aufgebauten Kammer vorgesehen, wobei der Stofftransport der präkonzentrierten flüchtigen organischen Stoffe zu den Chemiresistoren per Diffusion erfolgt.

Eine Messung mit einer solchen Anordnung erfordert eine Definition des Messprozesses, insbesondere der Wahl des Materials und des Volumens des Präkonzentrators, der Zeiten der Sorptionsphase und der Messphase sowie der Temperatur zur Freisetzung des sorbierten angereicherten Stoffes und dergleichen. Weiterhin hängen die Messergebnisse des Fluidsensors erheblich von Eigenschaften der unmittelbaren Umgebung des Präkonzentrators, d. h. des mikromechanischen Aufbaus des Detektors ab, die nur unzureichend durch ein physikalisches Modell beschrieben werden können.

Daher ist eine Kalibrierung bzw. eine Justage des Fluiddetektors notwendig, um der elektrischen Messgröße eine bestimmte Fluidkonzentration eines zu detektierenden Stoffes zuzuordnen. Eine solche Kalibrierung muss die aufgrund von Fertigungstoleranzen und Alterungseffekten variierenden Eigenschaften der Messstrecke, insbesondere bezogen auf den Präkonzentrator, ausgleichen.

Die Kalibrierung des Fluiddetektors zur Messung einer Konzentration des zu detektierenden Stoffes über eine Korrektur der Auswertung des Fluidsensorsignals ist grundsätzlich möglich, hat aber den Nachteil, dass sich dadurch die Unter- und Obergrenze des Messbereichs der zu detektierenden Konzentrationen verschiebt und somit für die Fluiddetektoren individuell abweichen.

Die Druckschrift LEIDINGER MARTIN ET AL, "Integrated pre-concentrator gas Sensor microsystem for ppb level benzene detection", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, Bd. 236, 14. April 2016, Seiten 988-996 offenbart ein Messsystem mit einem Fluiddetektor, der einen Präkonzentrator und einen Fluidsensor in einer durch eine Einlassöffnung zur Umgebung hin geöffneten Kammer aufweist, und mit einer Steuereinheit, die ausgebildet ist, um ein Messverfahren zum Detektieren einer Konzentration eines zu detektierenden Stoffes mit einer Sorptionsphase, während der das zu detektierende Fluid für eine Sorptionszeitdauer im Präkonzentrator in einem ersten Zustand angereichert wird, und einer Messphase auszuführen, in der eine durch Freigeben des im Präkonzentrator angereicherten zu detektierenden Stoffes resultierenden Konzentration des zu detektierenden Stoffes gemessen wird. Die Sorptionszeitdauer kann variieren.

Die Druckschrift JP2008-185446 offenbart ein Kalibrierverfahren einer Gaskonzentrationsmessvorrichtung, die einen Gassensor zur Ausgabe eines Signals proportional zur Konzentration des zu messenden Gases verwendet. Dabei wird ein Konzentrationsanzeigewert des zu messenden Gases zu einer unbekannten Konzentration des zu messenden Gases erfasst; das zu messende Gas nach Verdünnung in einem bekannten Verhältnis injiziert und ein Konzentrationsanzeigewert zu dieser Zeit erfasst. Es wird eine lineare Funktion aus jedem Konzentrationsanzeigewert und dem Verdünnungsverhältnis berechnet, und dieser korrigiert.

Es ist daher Aufgabe der vorliegenden Erfindung, ein Verfahren und eine Vorrichtung zum Kalibrieren oder Abgleichen eines Fluiddetektors mit einem Präkonzentrator zur Verfügung zu stellen, das eine höhere Genauigkeit des derartigen Fluiddetektors und einen stabilen Messbereich ermöglicht. Zudem soll eine Nachkalibrierung in einfacher Weise möglich sein.

Offenbarung der Erfindung

Diese Aufgabe wird durch das Verfahren zum Kalibrieren eines Fluiddetektors mit einem Präkonzentrator und einem Fluidsensor gemäß Anspruch 1 sowie durch die Vorrichtung gemäß dem nebengeordneten Anspruch gelöst.

Weitere Ausgestaltungen sind in den abhängigen Ansprüchen angegeben.

Gemäß einem ersten Aspekt ist ein Verfahren zum Kalibrieren eines Messsystems mit einem Fluiddetektor, der einen Präkonzentrator und einen Fluidsensor aufweist, wobei eine Konzentration eines zu detektierenden Stoffes mit einer Sorptionsphase, während der das zu detektierende Fluid für eine Sorptionszeitdauer im Präkonzentrator in einem ersten Zustand angereichert wird, und einer Messphase, in der eine durch Freigeben des im Präkonzentrator angereicherten zu detektierenden Stoffes resultierenden Konzentration des zu detektierenden Stoffes gemessen wird, detektiert wird, wobei das Messsystem durch Einstellen der Sorptionszeitdauer kalibriert wird.

Ein zu kalibrierender Fluiddetektor umfasst einen Präkonzentrator und einen in dessen Nähe angeordneten Fluidsensor zur Erfassung eines oder mehrerer zu detektierender Stoffe. Der Präkonzentrator ist selektiv für einen oder mehrere zu detektierende Stoffe und kann diese in einem ersten Zustand sorbieren und in einem zweiten Zustand freisetzen, um die Konzentration des einen oder der mehreren betreffenden Stoffe in der unmittelbaren Umgebung des Präkonzentrators zu erhöhen. Die Sorption auf dem Präkonzentrator kann sowohl eine Adsorption sein, also eine Anlagerung an die Oberfläche eines porösen Materials oder eine Absorption im Volumen des Präkonzentratormaterials.

Durch Diffusion gelangt der eine oder die mehreren betreffenden Stoffe in den Bereich des Fluidsensors. Der Fluidsensor kann eine Messung einer Konzentration oder einer Stoffmenge vornehmen und eine entsprechende elektrische Messgröße abhängig von der Konzentration der Stoffmenge bereitstellen. Der Präkonzentrator und der Fluidsensor sind in einer Kammer so angeordnet, dass die Einwirkung eines in der Umgebung befindlichen Fluids auf den Präkonzentrator über eine Diffusionsstrecke erfolgt, und die Diffusion des einen oder der mehreren betreffenden zu detektierenden Stoffe durch den Fluidsensor möglichst von der Konzentration der zu detektierenden Stoffe in der Detektorumgebung unabhängig ist.

Der Betrieb eines solchen Fluiddetektors zur Messung einer Konzentration der zu detektierenden Stoffe erfolgt in zwei Phasen. In einer Sorptionsphase wird der eine oder die mehreren zu detektierenden Stoffe in dem Präkonzentrator angereichert und in einer Messphase werden die angereicherten Stoffe freigesetzt, so dass diese in den Bereich des Fluidsensors gelangen, um dort eine entsprechende Messung deren Konzentration bzw. Stoffmenge vorzunehmen. Dadurch ergibt sich eine zusätzliche Selektivität des Detektors, die in der Regel in engen Grenzen spezifiziert werden muss. Abhängig von den Parametern des zyklischen Betriebs der durch den Sensoraufbau gegebenen physikalischen Gegebenheiten kann von der elektrischen Messgröße des Fluidsensors auf die Konzentration des einen oder der mehreren Stoffe in der Detektorumgebung geschlossen werden.

Der Fluidsensor liefert bei der Messung eine elektrische Messgröße, die von einer Konzentration des zu detektierenden Stoffes während der Messphase abhängt. Die Kalibrierung des Fluidsensors, d.h. deren Sensitivität als Quotient zwischen der gemessenen Konzentration und der elektrischen Messgröße wird in der Regel herstellungsseitig für eine Anzahl von Fluiddetektoren voreingestellt. Die Kalibrierung des Fluiddetektors zur Messung einer Konzentration des zu detektierenden Stoffes über eine Korrektur der Auswertung des Fluidsensorsignals ist grundsätzlich möglich, hat aber den Nachteil, dass sich dadurch die Unter- und Obergrenze des Messbereichs der zu detektierenden Konzentrationen verschieben und somit für die bereitgestellten Fluiddetektoren individuell abweichen. Außerdem hat dies den Nachteil, dass sich das Konzentrationsverhältnis zwischen Fluiden, die selektiv angereichert werden und Fluiden, die nicht selektiv angereichet werden, verändert und sich damit die Selektivität des Sensors ändert.

Es wird daher gemäß dem obigen Verfahren vorgeschlagen, eine Kalibrierung eines solchen Fluiddetektors vorzunehmen, indem die Zeitdauer der Anreicherung des zu detektierenden Stoffes in dem Präkonzentrator während der Sorptionsphase eingestellt wird.

Zur Beschreibung des physikalischen Verhaltens des Fluiddetektors können der Stofftransport zu dem Präkonzentrator und die Speicherfähigkeit im Präkonzentrator bestimmt werden. Die Anreicherungszeit kann dann entsprechend so eingestellt werden, dass ein über die Fluiddetektoren erfasste elektrische Messgröße in definierter Weise von der zu messenden Konzentration des zu detektierenden Stoffes abhängt.

Weiterhin kann das Verfahren folgende Schritte aufweisen:

- Bestimmen einer Speicherfähigkeit des Präkonzentrators in einem Gleichgewichtszustand in dem ersten Zustand;
- Ermitteln der Sorptionszeitdauer abhängig von der Speicherfähigkeit des Präkonzentrators und abhängig von einer Angabe zu einem vorgegebenen Verhältnis zwischen gespeicherten Stoffmenge und Konzentration des zu detektierenden Stoffes.

Insbesondere können die folgenden Schritte vorgesehen sein:

- Bestimmen einer im Präkonzentrator gespeicherten Stoffmenge in einem Nicht-Gleichgewichtszustand nach einer vorbestimmten Testzeitdauer;
- Ermitteln der Sorptionszeitdauer weiterhin abhängig von der bestimmten Stoffmenge.

Gemäß einer Ausführungsform kann die Speicherfähigkeit des Präkonzentrators eine maximal speicherbare Stoffmenge des zu detektierenden Stoffes für eine bestimmte Temperatur und für eine bestimmte Konzentration des zu detektierenden Stoffes angeben und durch ein Verfahren zur Bestimmung einer im Präkonzentrator gespeicherten Stoffmenge, insbesondere durch ein DSC-Verfahren, werden.

Es kann vorgesehen sein, dass die dynamische Aufnahme des zu detektierenden Stoffes im Präkonzentrator durch eine Differentialgleichung erster Ordnung beschrieben wird.

Es kann eine Vorrichtung zum Kalibrieren eines Messverfahrens für ein Messsystem mit einem Fluiddetektor vorgesehen sein, der einen Präkonzentrator und einen Fluidsensor aufweist, wobei das Messverfahren zum Detektieren einer Konzentration eines zu detektierenden Stoffes mit einer Sorptionsphase, während der das zu detektierende Fluid für eine Sorptionszeitdauer im Präkonzentrator in einem ersten Zustand angereichert wird, und einer Messphase, in der eine durch Freigeben des im Präkonzentrator angereicherten zu detektierenden Stoffes resultierenden Konzentration des zu detektierenden Stoffes gemessen wird, ausgebildet ist, wobei die Vorrichtung ausgebildet ist, den Fluiddetektor durch Einstellen der Sorptionszeitdauer zu kalibrieren.

Gemäß einem weiteren Aspekt ist ein Messsystem mit einem Fluiddetektor, der einen Präkonzentrator und einen Fluidsensor in einer durch eine Einlassöffnung zur Umgebung hin geöffneten Kammer aufweist, und mit einer Steuereinheit vorgesehen, die ausgebildet ist, um eine Konzentration eines zu detektierenden Stoffes mit einer Sorptionsphase, während der das zu detektierende Fluid für eine Sorptionszeitdauer im Präkonzentrator in einem ersten Zustand angereichert wird, und einer Messphase zu detektieren, in der eine durch Freigeben des im Präkonzentrator angereicherten zu detektierenden Stoffes resultierenden Konzentration des zu detektierenden Stoffes gemessen wird, wobei die Steuereinheit ausgebildet ist, um das Messsystem durch variables Einstellen der Sorptionszeitdauer zu kalibrieren.

Kurzbeschreibung der Zeichnungen

Ausführungsformen werden nachfolgend anhand der beigefügten Zeichnungen näher erläutert. Es zeigen:

Figur 1    eine schematische Darstellung eines Fluiddetektors in mikromechanischer Ausfertigung;

Figur 2    ein Flussdiagramm zur Veranschaulichung des Verfahrens zur Kalibrierung des Fluiddetektors.

Beschreibung von Ausführungsformen

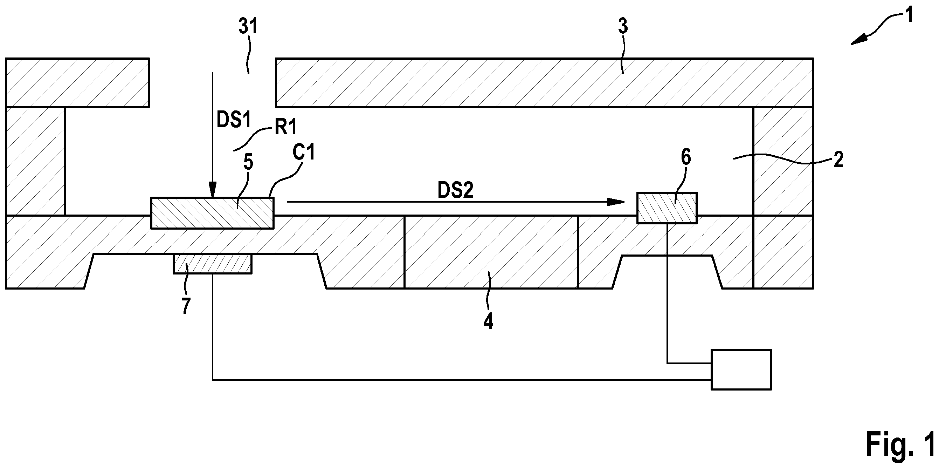

Figur 1 zeigt eine schematische Darstellung eines Fluiddetektors 1 in mikromechanischer Ausfertigung. Der Fluiddetektor 1 dient zur Detektion eines zu detektierenden Stoffes in einem Trägerfluid. Der Fluiddetektor 1 kann als Gasdetektor ausgebildet sein, der eine Konzentration von sogenannten VOC (flüchtige organische Substanzen) misst. Alternativ kann der Fluiddetektor auch Konzentrationen von flüssigen Stoffen in einer Trägerflüssigkeit messen.

Der Fluiddetektor 1 umfasst eine Kammer 2, die beispielsweise zwischen zwei flächigen Substraten 3, 4 angeordnet sein kann. Die Substrate 3, 4 können Glassubstrate, Siliziumsubstrate oder allgemein Substrate sein, die sich für eine mikromechanische Strukturierung eignen. Eines der Substrate 3, das abgesehen von einer Einlassöffnung nicht weiter strukturiert wird, kann auch aus einem anderen Material, wie z.B. einem Metallmaterial ausgebildet sein.

Eines der Substrate 4 trägt einen Präkonzentrator 5 und einen dazu benachbarten für einen zu detektierenden Stoff in der Kammer 2 sensitiven Fluidsensor 6. Das gegenüberliegende Substrat 3 verschließt die Kammer 2, wobei nahe des Präkonzentrators 5 insbesondere dem Präkonzentrator 5 gegenüberliegend eine Einlassöffnung 31 vorgesehen sein kann, um eine durchlässige Verbindung zwischen dem Umgebungsfluid und dem Fluid in der Kammer 2 des Fluiddetektors 1 vorzusehen.

Der Präkonzentrator 5 ist mit einer Heizeinrichtung 7 so gekoppelt, dass dieser von einem ersten Zustand, beispielsweise einem Zustand niedriger Temperatur, in einen zweiten Zustand, z. B. einen Zustand hoher Temperatur, gebracht werden kann. Die Heizeinrichtung 7 kann außenseitig des Substrats 4 dem Präkonzentrator 5 gegenüberliegend angeordnet sein, um durch die Heizeinrichtung 7 generierte Wärme möglichst schnell und direkt dem Präkonzentrator 5 zuzuführen. Die Heizeinrichtung 7 dient dazu, den Präkonzentrator 5 von einem ersten Zustand einer ersten Temperatur zu einem zweiten Zustand einer zweiten Temperatur zu bringen. Alternativ kann die Zustandsänderung auch durch andere Mittel wie beispielsweise einem Anlegen eines elektrischen oder magnetischen Feldes oder dergleichen bewirkt werden.

Der Präkonzentrator 5 kann ein homogenes Material, z. B. eine hochviskose Flüssigkeit, wie PMDS, oder ein mikroporöses oder mesoporöses Material sein oder enthalten (z. B. ein metallorganisches Netzwerk (MOF)). Material und Struktur des Präkonzentrators 5 können gewählt sein, um einen oder mehrere bestimmte, zu detektierende Stoffe zu sorbieren und/oder einen oder mehrere bestimmte von der Detektion auszuschließende Stoffe nicht zu sorbieren.

In dem ersten Zustand ist der Präkonzentrator 5 in der Lage, ein oder mehrere bestimmte zu detektierende flüchtige organische Stoffe zu sorbieren und anzureichern. Die Speicherfähigkeit des Präkonzentrators 5 hängt von dessen Temperatur ab. Daher kann der Präkonzentrator 5 aufgrund der reduzierten Speicherfähigkeit im Gleichgewichtszustand im zweiten Zustand (höhere Temperatur) den einen oder die mehreren angereicherten zu detektierenden Stoffe in die unmittelbare Umgebung des Präkonzentrators 5, d. h. in die Kammer 2 abgeben, so dass der zu detektierende Stoff durch das an sich unbewegliche (strömungslose) Fluid in der Kammer 2 in Richtung des Fluidsensors 6 diffundieren kann.

Der Fluidsensor 6 kann als halbleitender Metalloxidsensor, als organischer Halbleitersensor, als gassensitiver FET (Gas-FET) oder dergleichen ausgeführt sein. In der Regel kann mithilfe des Fluidsensors 6 eine elektrische Messgröße, insbesondere ein elektrischer Widerstand, abhängig von einer Fluidkonzentration des zu detektierenden Stoffes und/oder abhängig von einer während eines Messzeitfensters auf den Fluidsensor 6 einwirkenden Stoffmenge (Dosis) erfasst und in einer Steuereinheit 10 ausgewertet werden.

Die Heizeinrichtung 7 zum Aufheizen des Präkonzentrators 5 kann ein Widerstandsleiter mit einem bekannten temperaturabhängigen Widerstand sein, wie beispielsweise aus Platin, so dass neben der Heizwirkung durch Beaufschlagung mit elektrischer Leistung eine Temperaturmessung über dem elektrischen Widerstand des Heizleiters vorgenommen werden kann.

Der Fluiddetektor 1 wird im Messbetrieb mithilfe einer Steuereinheit 10 betrieben. Die Steuereinheit 10 steuert dazu den Fluiddetektor 1 in einem zyklischen Betrieb mit einer Sorptionsphase und einer Messphase an. In der Sorptionsphase gelangt der zu detektierende Stoff über die Einlassöffnung 31 zu dem Präkonzentrator 5 für eine vorbestimmte Sorptionzeitdauer und wird dort sorbiert. Die Sorptionzeitdauer bestimmt sich als Zeitraum zwischen einem Ende einer vorausgegangenen Messphase, zu dem der Präkonzentrator 5 von der zweiten Temperatur auf die erste Temperatur abgekühlt wird, und dem Startzeitpunkt einer darauffolgenden Messphase, ab der der Präkonzentrator 5 von der ersten Temperatur auf die zweite Temperatur aufgeheizt wird.

Durch Festlegen der Sorptionzeitdauer der Sorptionsphase wird die eingelagerte Stoffmenge abhängig von deren Konzentration im Umgebungsfluid festgelegt. In der Messphase wird nach einer vorbestimmten Messzeitdauer nach dem Ende der Sorptionsphase eine Konzentration bzw. Stoffmenge gemessen. Ein definierte Fluidsensorkennlinie vorausgesetzt erhält man ein auswertbares Messsignal bei konstanter Messzeitdauer.

Zur Kalibrierung des Messverfahrens mit dem Fluiddetektor wird nun die Sorptionszeitdauer angepasst und zur Durchführung des Messverfahrens festgelegt.

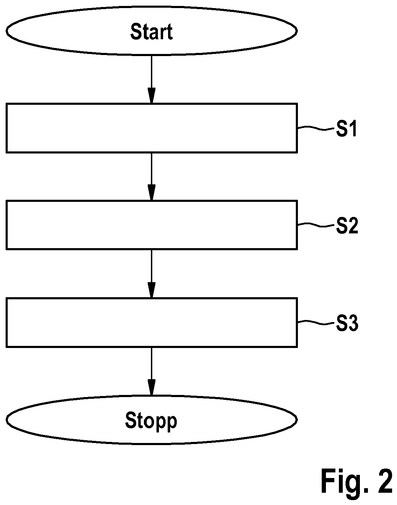

Das Kalibrierungsverfahren wird anhand des Flussdiagramms der Figur 2 näher erläutert.

Der Transport des zu detektierenden Stoffes in und innerhalb des Fluiddetektors 1 wird aufgrund der geringeren Abmessungen fast vollständig durch Diffusion bestimmt und kann daher mithilfe der Fick'schen Gesetze beschrieben werden. Da die Lösung des allgemeinen Falles (zweites Fick'sches Gesetz) in der Regel nicht analytisch möglich ist und die Ergebnisse nicht anschaulich sind, wird eine zulässige Vereinfachung der Beschreibung auf stationäre Fälle verwendet. Insbesondere im zeitlichen Bereich des Übergangs von Sorptions- und Messphase kann diese Vereinfachung ggfs. nicht angebracht sein. In diesem Fall kann das System durch Näherungslösungen des allgemeinen Falles ergänzt werden. Für das Kalibrierungsverfahren ist es wichtig, dass der Prozess in einen stationären Zustand übergegangen ist. Dies kann durch die Formel

$$L = \sqrt{Dt} \left( t \geq \frac{L^2}{D} \right)$$

abgeschätzt werden. Dabei ist D die Diffusionskonstante des Gases, L die charakteristische Länge der Diffusionsstrecke und t die Zeit, bis eine Diffusion über eine Strecke L erfolgt, d.h. die Diffusionsfront die Diffusionsstrecke L zurückgelegt hat.

Zum Einstellen der Detektorcharakteristik, d. h. der Sensitivität in Form eines Verhältnisses zwischen der zu messenden Konzentration bzw. Stoffmenge des zu detektierenden Stoffes und der resultierenden elektrischen Messgröße, wird die Sorptionszeitdauer so eingestellt, dass diese einen vorbestimmten Wert des Quotienten zwischen sorbierter Stoffmenge und der zu messenden Konzentration aufweist. Dies ermöglicht eine lineare Abhängigkeit der durch den Fluidsensor 6 zu messenden angereicherten Konzentration in der Messphase von der zu messenden Konzentration.

Hierzu wird in Schritt S1 die Speicherfähigkeit C1 des Präkonzentrators 5 ermittelt, indem ein Testfluid mit einer vorgegebenen Konzentration eines oder mehreren zu detektierenden Stoffen appliziert wird. Die Speicherfähigkeit C1 des Präkonzentrators 5 entspricht einer Stoffmenge n, die im Präkonzentrator 5 bei einer bestimmten Konzentration $c_0$ des Testfluids im stationären Fall (d.h. bei Sättigung bzw. im Gleichgewichtszustand) bei der ersten Temperatur aufgenommen wird. Die im Präkonzentrator 5 gespeicherte Stoffmenge n ist proportional zur sorbierten Stoffmenge C1 des zu detektierenden Stoffes.

Diese Stoffmenge $n_{PC}$ kann auf verschiedene Arten gemessen werden. Die sorbierte Stoffmenge $n_{PC}$ des zu detektierenden Stoffes ist im stationären Fall proportional zur Konzentration $c_0$ des zu detektierenden Stoffes, zu dem Verteilkoeffizienten k und zu dem Volumen $V_{PC}$ des Präkonzentrators 5 gemäß der Formel

$$n_{PC} = c_0 * k * V_{PC} = c_0 * C1$$

Zur Bestimmung der sorbierten Stoffmenge n des zu detektierenden Stoffes im Testfluid wird der Präkonzentrator 5 mit dem Testfluid einer bekannten Konzentration bis zu einer Einstellung eines Gleichgewichts belegt. Die hierfür angenommene Zeitdauer sollte mindestens $5\tau_{med}$ betragen, die mit

$$\tau_{med} = \frac{1}{R_{1med} * C_{1med}}$$

anfänglich geschätzt werden kann, wobei $\tau_{med}$, $R_{1med}$, $C_{1med}$ die im Median vorkommenden Ausprägungen der Größen $\tau$, $R_1$, $C_1$ sind. Für die Speicherfähigkeit gilt:

$$C_1 := k * V_{PC} = \frac{n_{PC}(t \to \infty)}{c_0}$$

Beispielsweise kann mithilfe eines an sich bekannten DSC-Verfahrens die im Präkonzentrator 5 gespeicherte Stoffmenge $n_{PC}$ ermittelt werden. Dazu wird eine Heizleistung des belegten Präkonzentrators 5 bei einer vorgegebenen Temperaturrampe gemessen. Die Temperaturrampe kann durch Anlegen einer vorgegebenen elektrischen Leistung insbesondere durch Vorgeben eines Verlaufs an zugeführter elektrischer Leistung bestimmt sein. Die Differenz der Energie, die anhand des Integrals der Heizleistung während der Messdauer bestimmt wird, ist näherungsweise einem Produkt aus der molaren Verdampfungsenergie, die für den zu detektierenden Stoff bzw. für die zu detektierenden Stoffe vorgegeben ist, und der Stoffmenge des verdampften zu detektierenden Stoffes. Die gemessene Heizleistung kann mithilfe einer Referenzkurve für einen Heizer ohne Präkonzentrator 5 kompensiert werden, wobei die kompensierte Heizleistung der Wärmekapazität des Präkonzentrators 5 entspricht. Im Falle eines Phasenübergangs des zu detektierenden Stoffes ist die Energie für den entsprechenden Phasenübergang zu berücksichtigen.

Auch andere Verfahren zur Messung der gespeicherten Stoffmenge sind denkbar, wie z.B. basierend auf einer Auswertung einer Konzentrationsänderung im Testfluid bei vorgegebenem Volumen, Auswertung gravimetrischer Änderungen, Massedifferenzbestimmung einer Präkonzentratormaterialprobe durch Schwingungssysteme und dergleichen.

Zum Beschreiben des dynamischen Verhaltens des Fluiddetektors 1 ist eine Beschreibung des Stofftransports über die Diffusionsstrecke von der Einlassöffnung 31 zum Präkonzentrator 5 während der Sorptionsphase notwendig. Dazu wird in Schritt S2 ein Zutrittswiderstand R1 des zu detektierenden Stoffes zum Präkonzentrator 5 bestimmt. Der Zutrittswiderstand R1 des zu detektierenden Stoffes durch die Einlassöffnung zu dem Präkonzentrator 5 ist maßgeblich durch die geometrische Struktur des Fluiddetektors 1 bestimmt. Der Zutrittswiderstand R1 entspricht einem Diffusionsbarrierewert und stellt jeweils einen Quotienten aus Diffusionsweg $x_D$ und dem Produkt aus Diffusionskoeffizient D in dem Trägermedium und einer Diffusionsfläche A dar:

$$R1 = \frac{x_D}{D * A}$$

Zur Bestimmung der zeitlichen Änderung der Stoffmenge im Präkonzentrator 5 während der Sorptionsphase wird eine Differentialgleichung erster Ordnung zugrunde gelegt.

$$c(t) = R_1 \frac{dn_{PC}}{dt} + \frac{n_{PC}}{C_1}$$

Die analytisch errechnete allgemeine Lösung für die Differentialgleichung für die Stoffmenge in dem Präkonzentrator 5 gilt:

$$n_{PC}(t) = c_0 * k * V_{PC}\left(1 - e^{-\left(\frac{t * D_{air} * A}{x_D * k * V_{PC}}\right)}\right)$$

$$n_{PC}(t) = c_0 * C_1 \left(1 - e^{-\left(\frac{t}{R_1 * C_1}\right)}\right)$$

Somit lässt sich durch eine Messung der im Präkonzentrator 5 gespeicherten Stoffmenge $n_{PC}(t_1)$ nach einer Zeitdauer $t_1$ nach Beginn eines Anlegens eines Testfluids der Zutrittswiderstand R1 bestimmen mit:

$$R_1 = - \frac{t_1}{C_1 ln(1 - \frac{n_{PC}(t_1)}{c_0 * C_1})}$$

Dabei sollte die Zeitdauer $t_1 < 2\tau$ gewählt werden.

In Schritt S3 wird nun mit bekanntem C1 und R1 und mit einem für den Fluiddetektor 1 vorgegebenen Quotienten $\frac{n_{PC}}{c_0}$ aus Stoffmenge $n_{PC}$ und Konzentration des zu detektierenden Stoffes $c_0$ die einzustellende Sorptionszeitdauer $\bar{t}$ bestimmt

$$\bar{t} = - R_1 * C_1 * ln(1 - \frac{\frac{n_{PC}}{c_0}}{C_1})$$

Diese Bestimmung der Sorptionszeitdauer $\bar{t}$ und deren Anwendung in einem Messvorgang ermöglicht einen Ausgleich von Fertigungstoleranzen und Eigenschaftsänderungen durch Alterung. Insbesondere ist kein Eingriff in die elektronische Signalauswertung notwendig, so dass der gesamte Messbereich erhalten werden kann.

**Patentansprüche**

1. Verfahren zum Kalibrieren eines Messsystems mit einem Fluiddetektor (1), der einen Präkonzentrator (5) und einen Fluidsensor (6) aufweist, wobei eine Konzentration eines zu detektierenden Stoffes mit einer Sorptionsphase, während der das zu detektierende Fluid für eine Sorptionszeitdauer (1) im Präkonzentrator (5) in einem ersten Zustand angereichert wird, und einer Messphase, in der eine durch Freigeben des im Präkonzentrator (5) angereicherten zu detektierenden Stoffes resultierende Konzentration des zu detektierenden Stoffes gemessen wird, detektiert wird, wobei das Messsystem durch Einstellen der Sorptionszeitdauer ($\bar{t}$) kalibriert wird.

2. Verfahren nach Anspruch 1, mit folgenden Schritten:

   - Bestimmen einer Speicherfähigkeit des Präkonzentrators (5) in einem Gleichgewichtszustand in dem ersten Zustand;
   - Ermitteln der Sorptionszeitdauer ($\bar{t}$) abhängig von der Speicherfähigkeit des Präkonzentrators (5) und abhängig von einer Angabe zu einem vorgegebenen Verhältnis zwischen gespeicherter Stoffmenge und Konzentration des zu detektierenden Stoffes.

3. Verfahren nach Anspruch 2, mit folgenden Schritten:

   - Bestimmen einer im Präkonzentrator (5) gespeicherten Stoffmenge in einem Nicht-Gleichgewichtszustand nach einer vorbestimmten Testzeitdauer;
   - Ermitteln der Sorptionszeitdauer ($\bar{t}$) weiterhin abhängig von der bestimmten Stoffmenge.

4. Verfahren nach einem der Ansprüche 1 bis 3, wobei die Speicherfähigkeit des Präkonzentrators (5) eine maximal speicherbare Stoffmenge des zu detektierenden Stoffes für eine bestimmte Temperatur und für eine bestimmte Konzentration des zu detektierenden Stoffes angibt und durch ein Verfahren zur Bestimmung einer im Präkonzentrator (5) gespeicherten Stoffmenge, insbesondere durch ein DSC-Verfahren bestimmt wird.

5. Verfahren nach einem der Ansprüche 1 bis 4, wobei die dynamische Aufnahme des zu detektierenden Stoffes im Präkonzentrator (5) durch eine Differentialgleichung erster Ordnung beschrieben wird.

6. Verfahren nach einem der Ansprüche 1 bis 5, wobei das Messverfahren mit einer Sorptionsphase für die Sorptionszeitdauer ($\bar{t}$) durchgeführt wird.

7. Messsystem mit einem Fluiddetektor (1), der einen Präkonzentrator (5) und einen Fluidsensor (6) in einer durch eine Einlassöffnung zur Umgebung hin geöffneten Kammer aufweist, und mit einer Steuereinheit (10), die ausgebildet ist, um eine Konzentration eines zu detektierenden Stoffes mit einer Sorptionsphase, während der das zu detektierende Fluid für eine Sorptionszeitdauer ($\bar{t}$) im Präkonzentrator (5) in einem ersten Zustand angereichert wird, und einer Messphase zu detektieren, in der eine durch Freigeben des im Präkonzentrator (5) angereicherten zu detektierenden Stoffes resultierenden Konzentration des zu detektierenden Stoffes gemessen wird, und um das Messsystem durch variables Einstellen der Sorptionszeitdauer ($\bar{t}$) zu kalibrieren.

**Claims**

1. A method of calibrating a measuring system comprising a fluid detector (1) having a preconcentrator (5) and a fluid sensor (6), wherein a concentration of a substance to be detected is measured at a sorption phase during which the fluid to be detected is enriched in the preconcentrator (5) in a first state for a sorption time period ($\overline{t}$), and at a measuring phase in which a concentration of the substance to be detected resulting from release of the substance to be detected enriched in the preconcentrator (5) is measured, wherein the measuring system is calibrated by adjusting the sorption time period ($\overline{t}$).

2. The method of claim 1, comprising the steps of:

- Determining a storage capacity of the preconcentrator (5) in an equilibrium state in the first state;
- Determining the sorption time duration ($\overline{t}$) depending on the storage capacity of the preconcentrator (5) and depending on an indication of a predetermined ratio between the stored amount of substance and the concentration of the substance to be detected.

3. Method according to claim 2, comprising the following steps:

- Determining an amount of substance stored in the preconcentrator (5) in a non-equilibrium state after a predetermined test time period;
- Determining the sorption time duration ($\overline{t}$) further depending on the determined amount of substance.

4. Method according to any one of claims 1 to 3, wherein the storage capacity of the preconcentrator (5) indicates a maximum storable amount of the substance to be detected for a certain temperature and for a certain concentration of the substance to be detected and is determined by a method for determining an amount of substance stored in the preconcentrator (5), in particular by a DSC method.

5. Method according to any one of claims 1 to 4, wherein the dynamic sorbation of the substance to be detected in the preconcentrator (5) is described by a first-order differential equation.

6. Method according to any one of claims 1 to 5, wherein the measurement method is carried out with a sorption phase for the sorption time period ($\overline{t}$).

7. A measuring system comprising a fluid detector (1) having a preconcentrator (5) and a fluid sensor (6) in a chamber opened to the environment through an inlet opening, and a control unit (10) adapted to measure a concentration of a substance to be detected at a sorption phase, during which the fluid to be detected is enriched in a first state in the preconcentrator (5) for a sorption time period ($\overline{t}$), and at a measurement phase in which a concentration of the substance to be detected resulting from release of the substance to be detected enriched in the preconcentrator (5) is measured, and to calibrate the measurement system by variably adjusting the sorption time period ($\overline{t}$).

**Revendications**

1. Procédé de calibrer d'un système de mesure comprenant un détecteur de fluide (1) comportant un préconcentrateur (5) et un capteur de fluide (6), dans lequel une concentration d'une substance à détecter est déterminée à une phase de sorption pendant laquelle le fluide à détecter est enrichi dans le préconcentrateur (5) dans un premier état pendant une durée de sorption ($\overline{t}$), et à une phase de mesure au cours de laquelle on mesure une concentration de la substance à détecter résultant de la libération de la substance à détecter enrichie dans le préconcentrateur (5), le système de mesure étant étalonné en réglant la durée de sorption ($\overline{t}$).

2. Procédé selon la revendication 1, comprenant les étapes suivantes :

- Déterminer une capacité de stockage du préconcentrateur (5) dans un état d'équilibre dans le premier état ;
- Déterminer la durée de sorption ($\overline{t}$) en fonction de la capacité de stockage du préconcentrateur (5) et en fonction d'une indication relative à un rapport prédéterminé entre la quantité de substance stockée et la concentration de la substance à détecter.

3. Procédé selon la revendication 2, comprenant les étapes suivantes :

- Détermination d'une quantité de substance stockée dans le préconcentrateur (5) dans un état hors équilibre après une durée de test prédéterminée ;
- Détermination de la durée de sorption ($\overline{t}$) en outre en fonction de la quantité de substance déterminée.

4. Procédé selon l'une des revendications 1 à 3, dans lequel la capacité de stockage du préconcentrateur (5) indique une quantité maximale de matière à stocker de la matière à détecter pour une température donnée et pour une concentration donnée de la matière à détecter et est déterminée par un procédé de détermination d'une quantité de matière stockée dans le préconcentrateur (5), notamment par un procédé DSC.

**5.** Procédé selon l'une des revendications 1 à 4, dans lequel le sorption dynamique de la substance à détecter dans le préconcentrateur (5) est décrit par une équation différentielle du premier ordre.

**6.** Procédé selon l'une des revendications 1 à 5, dans lequel le procédé de mesure est mis en oeuvre avec une phase de sorption pour la durée de sorption ($\bar{t}$).

**7.** Système de mesure comprenant un détecteur de fluide (1) qui présente un préconcentrateur (5) et un capteur de fluide (6) dans une chambre ouverte sur l'environnement par une ouverture d'entrée, et comprenant une unité de commande (10) qui est conçue pour déterminer une concentration d'une substance à détecter à une phase de sorption, pendant laquelle le fluide à détecter est enrichi dans un premier état pendant une durée de sorption ($\bar{t}$) dans le préconcentrateur (5), et à une phase de mesure dans laquelle une concentration de la substance à détecter résultant de la libération de la substance à détecter enrichie dans le préconcentrateur (5) est mesurée, et pour calibrer le système de mesure en réglant de manière variable la durée de sorption ($\bar{t}$).

Fig. 1

**Fig. 2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6902701 B1

- JP 2008185446 A

**In der Beschreibung aufgeführte Nicht-Patentliteratur**

- Integrated pre-concentrator gas Sensor microsystem for ppb level benzene detection. **LEIDINGER MARTIN et al.** SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS. ELSEVIER BV, 14. April 2016, 988-996